# EUROPEAN PATENT APPLICATION

(11) **EP 1 862 153 A1**
(43) Date of publication of application: **05.12.2007**
(21) Application number: 06730537.5
(22) Date of filing: 23.03.2006
(51) Int. Cl.: A61F 9/007

(54) **OPHTHALMIC CANNULA**

(30) Priority: 23.03.2005 JP 2005083766
(71) Applicant: Kanazawa University Technology Licensing Organization Ltd., Kanazawa shi, Ishikawa 920-1192 (JP)
(72) Inventor: NISHIMURA, Akira, Kanazawa-shi, Ishikawa, 9201192 (JP)
(74) Representative: Weber, Thomas
(86) International application number: PCT/JP2006/306589
(87) International publication number: WO 2006/101259

(57) **Abstract**

A configuration wherein the tip portion 1a of the cylindrical body 1, which constitutes the main body of the cannula, is closed with the membrane 2 made of a polymer material having elasticity and flexibility. Preferably, the cylindrical body 1 and the membrane 2 are integrally molded with the same membrane material. Thereby, the fluids in the eyeball are prevented from coming off through the cannula even when an instrument is removed. Also, provided that the cylindrical body 1 is made to have a partially swollen fusiform shape, a curved-tip instrument can be inserted. Also, provided that a projection is provided on the outer face of the cylindrical body, the cannula is prevented from coming off from the eyeball during surgery is prevented.

## Description

### Technical Field

The present invention relates to a cannula (also called a cannule) used in penetration into the eyeball for vitreous surgery and the like.

### Background Art

It is known that when a surgery involving insertion of one of various linear medical instruments into the eyeball, such as vitreous surgery, is performed, various complications can occur due to incarceration (impaction) of the vitreous into a penetrating hole formed in the eyeball wall for surgery (usually a scleral wound). For example, the occurrence of retinal detachment due to the formation of a retinal tear, prolonged vitreous hemorrhage due to anterior vitreous fibrovascular proliferation in diabetic retinopathy, and the like can be mentioned.

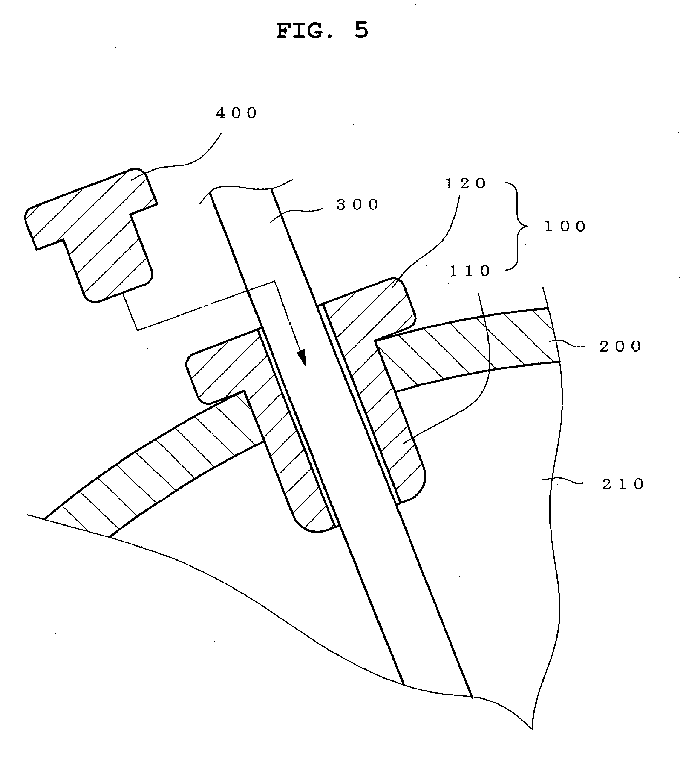

To suppress the above-described incarceration of the vitreous into a scleral wound, a metal cannula 100 is used as a guide pipe for instrument insertion, as shown in Figure 5, in inserting a medical instrument into the eyeball.

The cannula 100 consists of a cylindrical body 110 and a flange portion 120, the flange portion 120 serving as a stopper for defining the limit of the insertion of the cylindrical body 110 into the eyeball.

As such, the cannula 100 is used while penetrating the sclera 200, which constitutes the eyeball wall, and a medical instrument 300 such as a surgical knife is put in and out through the hollow portion of the cannula.

As examples of the cannula for vitreous surgery shown in Figure 5, a 19-gauge trocar cannula (made of metal material) manufactured by Grieshaber can be mentioned.

It has been reported that by using the cannula, compared to the case in which the cannula was not used, incarceration of the vitreous into a scleral wound was suppressed, and the occurrence of iatrogenic retinal tear around the scleral wound was suppressed, and the incidence of retinal detachment caused by the retinal tear decreased to one seventh (for example, Retina 17: 430-433. 1997, INFLUENCE OF THE CANNULATED VITRECTOMY SYSTEM ON THE OCCURENCE OF IATROGENIC SCLEROTOMY RETINAL TEARS.).

However, when the present inventor extensively examined the status of use of the above-described conventional cannula, it was found that the conventional cannula is useful in protecting the inside wall of the scleral wound, but has not solved the problem of the leakage of the fluid in the eyeball to the outside through the cannula upon removal of an instrument such as a vitreous cutter from the cannula.

Particularly, in the treatment performed with the eyeball filled with air, a plug 400 must be inserted into the cannula to close the pathway to prevent the air filling the intraocular space 210 of the eyeball from escaping to collapse the eyeball as soon as possible after the instrument 300 is removed from the cannula 100, as shown in Figure 5, which makes the work very difficult.

Also, because the conventional cannula has a direct tubular form, there is another problem wherein a curved-tip instrument such as vertical or horizontal scissors cannot be used because it is unable to pass the cannula.

It was also found that the cannula per se sometimes comes off from the wound hole during surgery. If the cannula comes off unintentionally, incarceration of the vitreous can occur.

### Disclosure of the Invention

It is an object of the present invention to solve the above-described problems, and to provide a cannula having a new configuration capable of preventing the fluid in the eyeball from escaping through the cannula even when the instrument is removed from the cannula during surgery.

It is another object of the present invention to confer a new configuration to a cannula so that even a curved-tip instrument can be inserted into the cannula, and to confer a new configuration to the cannula so that the cannula is more unlikely to come off from the wound hole.

The present invention has the following features:
(1) A cannula for use in penetration into the eyeball, having a cylindrical body penetrating the eyeball wall during use, wherein the tip portion of the cylindrical body is the tip portion of the cannula, wherein at least the tip portion of the cylindrical body is closed by a membrane made of a polymer material having elasticity and flexibility.
(2) The cannula for the eyeball, described in (1) above, wherein a cut penetrating in the direction of the thickness of the membrane made of a polymer material is provided in the membrane, this cut closes by itself due to the elasticity and flexibility of the polymer material, whereby the closure of the tip portion of the cylindrical body by the membrane is maintained.
(3) The cannula for the eyeball, described in (1) above, wherein the membrane is provided with a fragile portion along a line to be broken in the membrane made of a polymer material so that a medical instrument inserted into the cannula can easily pierce the membrane at the tip portion.
(4) The cannula for the eyeball, described in (1) above, wherein the cylindrical body is made of a polymer material having elasticity and flexibility, and the cylindrical body and the membrane closing the tip portion thereof are integrally formed with the same polymer material.
(5) The cannula for the eyeball, described in (1) above, wherein the cylindrical body is made of a polymer material having elasticity and flexibility, and the portion of the cylindrical body, entering the intraocular space upon paracentesis of the cannula into the eyeball, has a swollen fusiform shape.
(6) The cannula for the eyeball, described in (1) above, wherein the portion of the cylindrical body is provided with a projection on the surface thereof, entering the intraocular space, so that the cannula having punctured the eyeball can be prevented from coming off from the eyeball.
(7) The cannula for the eyeball, described in (6) above, wherein the cylindrical body is made of a polymer material having elasticity and flexibility, the portion of the cylindrical body, entering the intraocular space upon penetration of the cannula into the eyeball has a swollen fusiform shape, and the above-described projection is formed in the end portion on the base side of both end portions of this swell.
(8) The cannula for the eyeball, described in (1) above, wherein the base side of the cylindrical body is provided with a flange that defines the limit of insertion into the eyeball, and a projection around the outer periphery of the cylindrical body is provided on the outer face of the cylindrical body at a position apart by a distance not less than the thickness of the eyeball wall from the flange toward tip side, whereby a groove around the outer periphery of the cylindrical body is configured between the flange and the projection, so that upon paracentesis of the cannula into the eyeball, the eyeball wall surrounding the wound hole fits into the groove to prevent the cannula from coming off.
(9) The cannula for the eyeball, described in (8) above, wherein the cylindrical body is made of a polymer material having elasticity and flexibility, the portion of the cylindrical body, entering the intraocular space upon paracentesis of the cannula into the eyeball has a swollen fusiform shape, and the above-described projection is formed in the tip portion on the base side of both end portions of this swell.

### Brief Description of the Drawings

Figure 1 is a sectional view showing an example of the structure of the cannula according to the present invention, indicating a cylindrical body 1, a tip portion 1a, a membrane 2, a portion 3 for entry in the intraocular space, a swollen fusiform portion 4, a flange 5, a projection 6, and a groove 7.
Figure 2 is a sectional view showing an example embodiment of the tip portion of the cannula according to the present invention.
Figure 3 is an illustration showing an example embodiment of a membrane in the present invention.
Figure 4 is a schematic diagram showing the status of use of the cannula according to the present invention. In the drawing, the leader line of the groove 7 is drawn out from a portion of the inside wall of the groove in order to prevent the drawing from being made difficult to understand by the leader line of a symbol. In contrast, in Figure 1, a symbol 7 is given to indicate the width of the entire groove. The same applies to the flange 5.
Figure 5 is a schematic diagram showing the status of use of a conventional metal cannula.

### Best Mode for Embodying the Invention

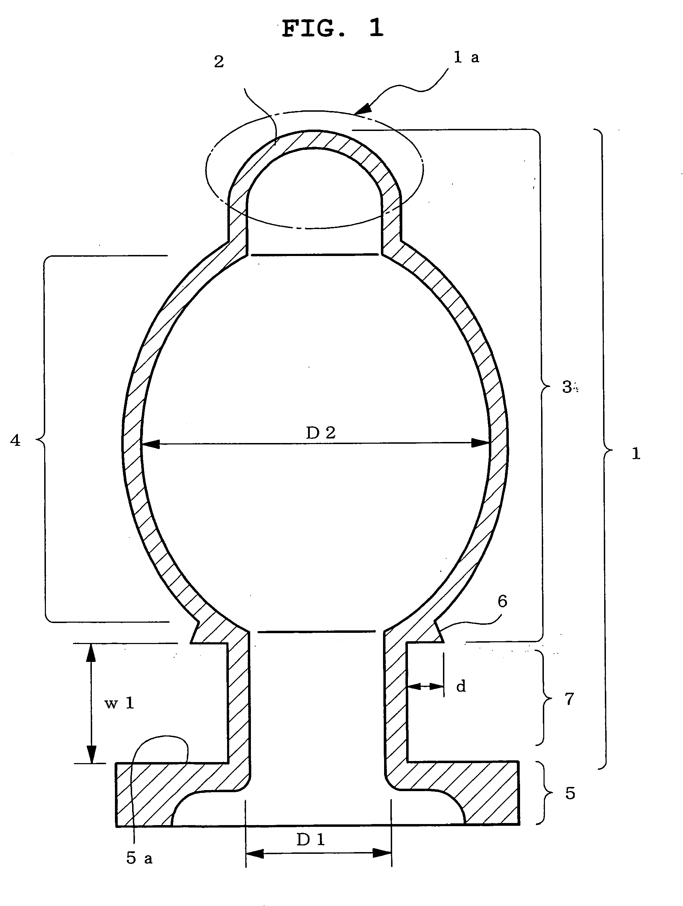

Figure 1 is a sectional view showing an example preferred embodiment of the cannula according to the present invention. As illustrated, the cannula has a cylindrical body 1. The tip portion 2 of this cylindrical body 1 is also the tip portion of the entire cannula. A feature of the cannula resides in that at least this tip portion 1a is closed by a membrane 2 made of a polymer material having elasticity and flexibility, as stated in the above-described effect of the invention.

The cannula for the eyeball of the present invention (hereinafter, cannula) has at least the tip portion (essentially, the tip portion that is open as a cylinder) 1a of the cylindrical body 1 closed with a membrane 2 made of a polymer material having elasticity and flexibility, as shown in Figure 1.

With this configuration, first, even if the cannula is penetrated through the eyeball wall (sclera), the inside fluid does not flow out through the cannula.

Second, during surgery, a cut can easily be made in the membrane 2 of the tip portion from the inside using a surgical knife and the like, and the necessary surgical instrument can be delivered while forcing a passage through this cut.

This cut of the membrane may be formed on the spot during surgery, and the cut may also be made in advance as designed. Even if such an existing cut is present, the cut closes itself by to the elasticity and flexibility of the polymer material of the membrane per se, so that the closure of the tip portion of the cannula is not hampered.

This configuration is most useful when the instrument inserted into the eyeball while forcing a passage through the cut is removed from the catheter. In the catheter, when the surgical instrument is removed, even without placing a plug as with the conventional catheter, the cut of the membrane closes itself by the elasticity and flexibility of the polymer material, so that the fluids in the eyeball, such as perfusion, are prevented from flowing out.

Particularly, in the case of a surgery involving air replacement, the collapse of the eyeball due to the leakage of the air in the eyeball can be prevented, so that the safety of the surgery increases, and time for plug insertion and the necessity for plug management can be obviated.

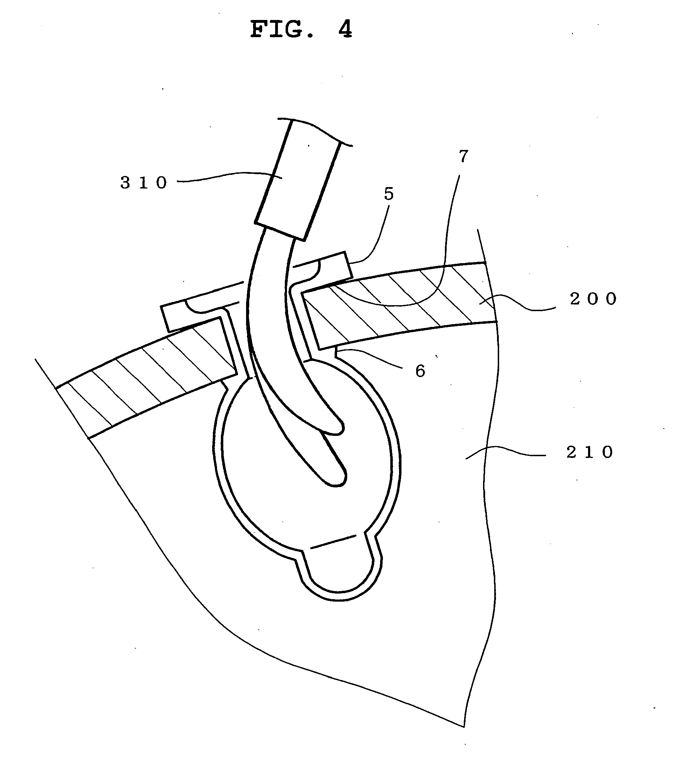

Also, by making the portion of the cylindrical body of the cannula, entering the intraocular space (particularly the portion indicated by symbol 4 in Figure 1), to have a swollen fusiform shape, and creating a locally wide space in the cannula, a curved-tip instrument 310 such as vertical or horizontal scissors can be inserted by means of the space, as shown in Figure 4.

Furthermore, as shown in Figure 1, by configuring a groove 7 around the outer periphery of the cylindrical body on the base side of the cylindrical body, the sclera 200 exposed in the wound hole gets impacted in this groove 7 upon placement of the cannula, as shown in Figure 4, whereby the cannula is prevented from coming off from the wound hole during surgery.

The full length of the cylindrical body is not subject to limitation, as long as it allows an application to the eyeball; for example, about 2 mm to 7 mm can be mentioned as a preferable size, in particular, in view of the ease of handling and the preventive effect on incarceration of the vitreous into the scleral wound, 4 mm to 6 mm is a more preferable size.

The basic inside diameter of the cylindrical body (excluding locally changing inside diameters such as the swollen fusiform portion in a preferred embodiment described below) is preferably a size that allows the insertion of various surgical instruments, and is as small as possible; for example, about 0.4 mm to 1.5 mm, in particular, 0.5 mm to 1.1 mm, can be mentioned as a more preferable size. These values represent a typical case; an appropriate size can be chosen as required.

The wall thickness of the cylindrical body, along with the material thereof, is described below.

The material of the membrane that closes at least the tip portion of the cannula must be a polymer material having elasticity and flexibility that meet the following requirements, as stated in the foregoing section of the effect of the invention:
(a) The material must have hardness and flexibility to the extent that a cut can easily be made in the membrane from the inside with an edge tool such as a surgical knife.
(b) Even if a cut is formed in the membrane in advance or on the spot during surgery, the material must have elasticity to the extent that the cut can close itself by the elasticity of the membrane per se when no medical instrument is penetrated.

A material that does not affect the body when inserted into the eyeball must be used.

Such a polymer material may be chosen as appropriate from among natural polymer materials (natural resins, natural rubber and the like) and synthetic polymer materials (synthetic resins, synthetic rubbers) that meet the requirements. Particularly, polymer materials in use for medical applications, such as soft polyvinyl chloride, polyolefin-series polymers (polypropylene, polyethylene and the like), polyethylene terephthalate, polyurethane, polycarbonate, silicone, and thermoplastic elastomers, are preferable elastic materials. Of them, silicone-based materials such as silicone resin and silicone rubber permit preparation and molding to have adequate flexibility and elasticity, and also have biocompatibility, and are therefore preferable materials.

To the polymer material, various additives and side-materials may be added as required.

The elasticity of the polymer material used in the membrane may be such that a restitutive force sufficient to allow the cut formed in the membrane to close itself can be produced, as stated above. For example, in the case of silicone rubber, one having a hardness of about 30 degrees to 80 degrees, as determined by the type A durometer test in JIS-K6253, exhibits preferable elasticity; for example, a hardness of 50 degrees is an example of preferable hardness. However, the foregoing range is not to be construed as limiting; a degree of hardness befitting the intended use and the like may be chosen.

Because membrane thickness, membrane shape and the like influence the restitutive force, it is preferable that these factors be considered in determining the elasticity of the polymer material.

Provided that the elasticity and flexibility of the material of the membrane are chosen in the above-described ranges, a preferable membrane thickness allowing the cut to close itself by the elasticity and flexibility thereof is 0.05 mm to 0.5 mm, in particular, 0.1 mm to 0.2 mm is a more preferable membrane thickness. However, these ranges only represent a preferred embodiment; a thicker membrane may be used as required.

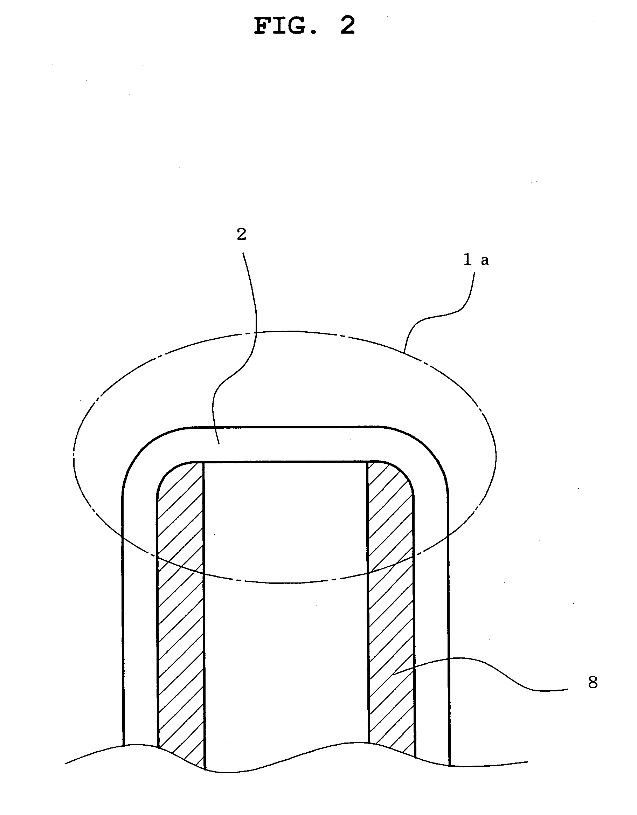

The embodiment of the membrane may be an outwardly convex sphere, as shown in Figure 1, a plane, as shown in Figure 2, and the like. For smooth insertion into the eyeball, the former is the preferable shape.

The material of the cylindrical body may be any material having rigidity to the extent of enduring insertion into the eyeball, and not affecting the body when inserted into the eyeball. For example, metal materials for use in common cannulas, such as stainless steel, titanium, and gold, the various polymer materials described above as membrane materials, and the like can be mentioned.

The wall thickness of the cylindrical body can be determined as appropriate according to the rigidity of the material, and about 0.05 mm to 0.5 mm, particularly about 0.05 mm to 0.2 mm, is a preferable range.

If the material of the cylindrical body and the material of the membrane of the tip portion are different, both may be joined as appropriate and, as exemplified in Figure 2, a multiple-layer structure having a layer 8 made of a rigid material and a layer 2 made of the material of the membrane may be formed to obtain a membrane having an extension of the layer 2 made of the material of the membrane.

Although the cannula is not subject to limitation, as long as at least the tip portion of the cylindrical body is closed by the above-described membrane made of a polymer material, it is preferable that the same polymer material as the material of the tip portion be used as the material of the cylindrical body to obtain a structure allowing both to be integrally molded, because the production is facilitated, and also because leak due to assembly errors and the like can be prevented. This also makes it possible to well fit the cannula into the wound hole without a gap by means of the elasticity of the cylindrical body.

If the cylindrical body and the membrane of the tip portion are integrally molded with the same polymer material, the thicknesses of individual portions, such as the wall thickness of the cylindrical body and the membrane thickness of the tip portion, may be independently determined as required.

Also, if the cylindrical body and the membrane of the tip portion are integrally molded, the composition of the material may be changed stepwise or continuously, from the cylindrical body to the tip portion, to confer rigidity, elasticity, and flexibility according to the function of each portion.

For the resin molding method and molding apparatus per se, commonly known technology may be referred to. Various procedures of mechanical processing such as cutting and welding, chemical processing and the like may be partially performed.

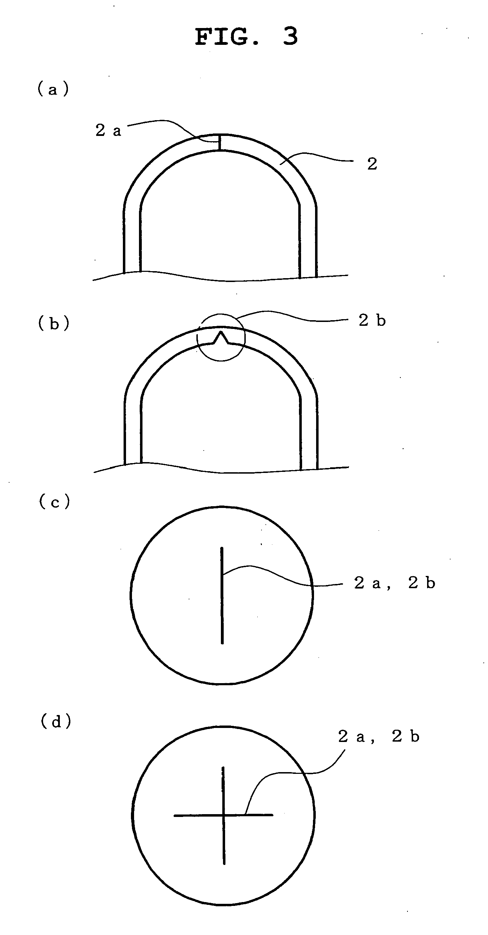

The membrane of the tip portion may be provided in advance with a cut 2a penetrating in the direction of the thickness of the membrane 2, as shown in Figure 3(a).

This cut is preferably substantially free from a gap, as with the cut obtained by making an incision using a surgical knife during surgery, and is preferably in a state wherein it closes itself by the elasticity and flexibility of the polymer material, whereby the closure of the tip of the cylindrical body by the membrane is maintained.

Another configuration is acceptable wherein in place of a cut, a fragile portion is previously given along a line to be broken, and the membrane is pierced easily or along an intended line by means of a medical instrument inserted into the cannula during use.

As examples of the embodiment of the fragile portion, an embodiment wherein a groove having a U-shaped section or a groove 2b having a V-shaped section is formed along a line to be broken on the inside face and/or outside face of the membrane to make the membrane to be easily breakable along the groove, as shown in Figure 3(b), an embodiment wherein a cut penetrating the membrane is formed in the form of a dotted line or a broken line to make the membrane to be easily breakable, an embodiment wherein the resin is modified along a line to be broken to make the membrane to be easily breakable, or an embodiment consisting of a combination thereof, and the like can be mentioned.

In the embodiment wherein the membrane is incised using a surgical knife during surgery, there is an advantage that the inside of the cannula can be kept completely closed until incision.

On the other hand, in the embodiment wherein a cut or a fragile portion is formed in the membrane in advance, the pattern of the line along which the membrane is broken can be freely controlled to make it easier to put in and out a surgical instrument, and also to allow more preferable closure after removal of the instrument. The fragile portion, in some embodiments, is also advantageous in that the inside of the cannula can be kept completely closed until incision.

As examples of the pattern of the line of the cut or fragile portion, a linear (curved along the curvature of the membrane) pattern, as shown in Figure 3(c), a cross pattern, as shown in Figure 3(d), a pentafurcate pattern or a radial pattern having a larger number of lines and the like can be mentioned. In Figures 3(c) and (d), for explanation, the lines of the cut 2a and the fragile portion 2b are drawn with solid lines.

As shown in Figure 1, by forming a cylindrical body with a polymer material, and making part or all of the portion 3 of the cylindrical body 1, entering the intraocular space, to have a swollen fusiform shape for both the inside diameter and the outside diameter, a curved instrument can be inserted by means of the inside space of the swollen portion. Also, because the cannula can be inserted into the eyeball while being folded (pressed inwardly) to reduce the outside diameter, the insertion work is facilitated.

The sizes and curvatures of the individual portions of this swollen fusiform portion may be determined as appropriate according to how the curved instrument is inserted. For example, the inside diameter of the most swollen portion is preferably about 1 mm to 3 mm, and the full length of the swollen portion is preferably about 1 mm to 6 mm.

For more preferably inserting the curved instrument, the starting position of the fusiform swell is preferably at a site of the cannula corresponding to the inside face of the eyeball wall (sclera).

In the embodiment in Figure 1, a linear cylindrical body is left on the tip side of the swollen fusiform portion 4. This is intended to stably support the inserted instrument using the inside wall face of the linear cylindrical body.

As shown in Figure 1, it is a preferable embodiment wherein a flange 5 is provided on the base side of the cylindrical body, whereby the limit of the insertion into the eyeball is defined by the flange face 5a.

The outside diameter of the flange may be larger by about 0.5 mm to 3 mm than the outside diameter of the cylindrical body.

The flange may be regarded as a member other than the cylindrical body, and may be regarded as a portion of the cylindrical body protruding from the outside face thereof.

As shown in Figure 1, by providing a projection 6 at any site (preferably a site further from the base side) of the portion 3 of the outer periphery face of the cylindrical body 1, entering the eyeball upon insertion of the cannula into the eyeball, the trouble wherein the cannula unintentionally comes off from the eyeball can be prevented as the projection serves as a checker.

The projection is preferably made in the form of what is called "a back rise" with a gradient on the tip side and a steeply rising level difference on the base side in order to reduce resistance during insertion into the eyeball, and to make the cannula to be less likely to come off from the eyeball after entry in the eyeball.

For making the cannula to be adequately unlikely to come off from the eyeball during surgery, and allowing the cannula to be relatively easily removed from the eyeball after surgery, the projection is preferably protruded in order to form a level difference of about 0.1 mm to 0.5 mm from the outer periphery of the cylindrical body.

The projection may be provided at a single point, may be provided at a plurality of points in the direction around the outer periphery of the cylindrical body, and may be a ridgeline projection in a circular form around the outer periphery of the cylindrical body.

If a swollen fusiform portion is provided in the cylindrical body, it is a preferable embodiment wherein a projection 6 is formed in the end portion on the base side of the swell, as shown in Figure 1.

If a flange 5 is provided on the base side of the cylindrical body, it is preferable that a projection 6 be formed at a position apart by a distance w1 not less than the thickness of the eyeball wall, from the flange face 5a toward the tip side, as shown in Figure 1. Thereby, relatively configured between the flange 5 and the projection 6 is a groove having the width w1 around the outer periphery of the cylindrical body.

By this groove, the eyeball wall gets impacted in the groove upon paracentesis of the cannula into the eyeball, so that the cannula is prevented from coming off from the wound hole during surgery, and from becoming unstable, as stated in the foregoing section of the effect of the invention.

This groove width w1 is preferably wider by about 0 mm to 0.3 mm than the thickness of the eyeball wall; because the thickness of the eyeball wall is about 0.5 mm to 1.0 mm when the eyeball wall is the sclera, the groove width w1 preferably has a value of about 0.5 mm to 1.3 mm.

Because the cannula has a flexible membrane in the tip portion thereof, it is preferable that a wound hole to which the cannula fits appropriately be formed using a tool for making a preparatory hole in advance, rather than allowing the cannula to pierce the eyeball wall by the rigidity thereof.

In inserting the cannula into the wound hole, a highly rigid mandrel may be inserted into the cannula, and the cannula may be inserted into the wound hole while being pushed out by the mandrel.

The cannula is useful in performing surgeries and examinations involving the insertion of one of various linear medical instruments into the eyeball, such as vitreous surgery and intraocular tissue biopsy.

### Examples

In this Example, a cannula in the embodiment shown in Figure 1 was prepared, and the status of use thereof was evaluated.

The cylindrical body and the membrane of the tip portion were integrally formed with the same silicone rubber. The sizes of the major portions are as follows:
Full length of cylindrical body 1 (excluding flange portion) = 4.2 mm
Reference inside diameter D1 of cylindrical body 1 = 0.9 mm
Full length of fusiform portion 4 = about 2.5 mm
Maximum inside diameter D2 of fusiform portion 4 = 2.1 mm
Mean wall thickness = 0.1 mm
Thickness of flange 5 = 0.4 mm
Outside diameter of flange 5 = 2.3 mm
Width w1 of groove 7 = 0.7 mm
Height d of projection 6 from surface of groove 7 = 0.2 mm
A wound hole 1.1 mm in opening diameter was formed in the sclera and the above-described cannula was inserted; it was found that the cannula was preferably fit by the elasticity of the material to close the wound hole and tightly seal the inside of the eyeball.

The membrane of the tip was incised using a 0.9 mm surgical knife, a 20-gauge vitreous cutter, scissors and the like were inserted, and vitreous surgery was performed, after which the cannula was removed and the wound was sutured. The wound hole remained intact and no incarceration of the vitreous was observed. Also, during the surgery, there was no trouble wherein the cannula unintentionally came off from the wound hole.

### Industrial Applicability

As stated above, by closing the tip portion of the cannula with a membrane made of a polymer material, it has become possible to prevent the fluids in the eyeball from coming off through the cannula even when an instrument is inserted into the cannula and then removed.

By providing a swollen portion in the cylindrical body to locally expanding the space in the cylinder, it has become possible to insert even a curved-tip instrument.

Furthermore, by providing a projection (relatively a groove) in the cylindrical body, it has become more unlikely that the cannula comes off from the wound hole.

This application is based on a patent application No. 2005-83766 filed in Japan, the contents of which are incorporated in full herein by this reference.

## Claims

1. A cannula for use in penetration into the eyeball,
having a cylindrical body penetrating the eyeball wall during use, wherein the tip portion of the cylindrical body is the tip portion of the cannula, wherein
at least the tip portion of the cylindrical body is closed by a membrane made of a polymer material having elasticity and flexibility.

2. The cannula of claim 1, wherein a cut penetrating in the direction of the thickness of the membrane made of a polymer material is provided in the membrane, this cut closes by itself due to the elasticity and flexibility of the polymer material, whereby the closure of the tip portion of the cylindrical body by the membrane is maintained.

3. The cannula of claim 1, wherein the membrane is provided with a fragile portion along a line to be broken in the membrane made of a polymer material so that a medical instrument inserted into the cannula can easily pierce the membrane at the tip portion.

4. The cannula of claim 1, wherein the cylindrical body is made of a polymer material having elasticity and flexibility, and the cylindrical body and the membrane closing the tip portion thereof are integrally formed with the same polymer material.

5. The cannula of claim 1, wherein the cylindrical body is made of a polymer material having elasticity and flexibility, and the portion of the cylindrical body, entering the intraocular space upon paracentesis of the cannula into the eyeball, has a swollen fusiform shape.

6. The cannula of claim 1, wherein the portion of the cylindrical body is provided with a projection on the surface thereof, entering the intraocular space, so that the cannula having punctured the eyeball can be prevented from coming off from the eyeball.

7. The cannula of claim 6, wherein the cylindrical body is made of a polymer material having elasticity and flexibility, the portion of the cylindrical body, entering the intraocular space upon penetration of the cannula into the eyeball has a swollen fusiform shape, and the above-described projection is formed in the end portion on the base side of both end portions of this swell.

8. The cannula of claim 1, wherein the base side of the cylindrical body is provided with a flange that defines the limit of insertion into the eyeball, and a projection around the outer periphery of the cylindrical body is provided on the outer face of the cylindrical body at a position apart by a distance not less than the thickness of the eyeball wall from the flange toward tip side, whereby a groove around the outer periphery of the cylindrical body is configured between the flange and the projection, so that upon paracentesis of the cannula into the eyeball, the eyeball wall surrounding the wound hole fits into the groove to prevent the cannula from coming off.

9. The cannula of claim 8, wherein the cylindrical body is made of a polymer material having elasticity and flexibility, the portion of the cylindrical body, entering the intraocular space upon paracentesis of the cannula into the eyeball has a swollen fusiform shape, and the above-described projection is formed in the tip portion on the base side of both end portions of this swell.
